# EUROPEAN PATENT APPLICATION

(11) **EP 4 660 197 A1**
(43) Date of publication of application: **10.12.2025**
(21) Application number: 24788225.1
(22) Date of filing: 12.04.2024
(51) Int. Cl.: C07D 498/14, C07D 471/14, A61K 31/53, A61K 31/5383, A61P 31/12, A61P 31/16

(54) **ACYL ETHYL ESTER POLYCYCLIC COMPOUND, AND PHARMACEUTICAL COMPOSITION AND USE THEREOF**

(30) Priority: 14.04.2023 CN 202310402118
(71) Applicant: Nanjing Cavendish Bio-Engineering Technology Co., Ltd., Nanjing, Jiangsu 210033 (CN)
(72) Inventor: YANG, Hao, Nanjing, Jiangsu 210033 (CN); XU, Yongxiang, Nanjing, Jiangsu 210033 (CN)
(74) Representative: Meissner Bolte Nürnberg
(86) International application number: PCT/CN2024/087551
(87) International publication number: WO 2024/213129

(57) **Abstract**

An acyl ethyl ester polycyclic compound, and a pharmaceutical composition and the use thereof. The acyl ethyl ester polycyclic compound is as represented by formula I, wherein X is S or Se. The compound has the effect of preventing or treating individuals infected by viruses.

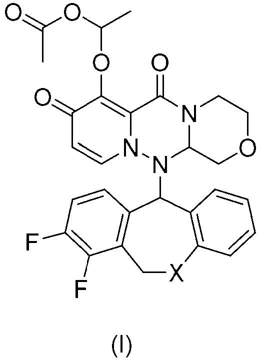

## Description

This present application claims the priority benefit of a Chinese invention patent application filed on April 14, 2023 with the invention title "Acyl Ethyl Ester Polycyclic Compound, and Pharmaceutical Composition Thereof and Use Thereof" and application number 202310402118.6, and the contents of which is incorporated herein by reference in their entirety.

### Technical Field

The present invention belongs to, but is not limited to, the technical field of medicine technology, specifically, an acyl ethyl ester polycyclic compound, and pharmaceutical composition thereof and use thereof.

### Background

Influenza, referred to simply as the flu, is an acute respiratory infectious disease caused by influenza virus. Influenza virus is the pathogen causing influenza, belonging to the *Orthomyxoviridae* family and is an RNA virus. At present, known small molecule anti-influenza virus drugs include neuraminidase inhibitor Oseltamivir, viral polymerase inhibitor Baloxavir Marboxil and M2 ion channel blocker Amantadine, etc.

Shionogi & Co., Ltd. disclosed Baloxavir, Baloxavir Marboxil and Compound 11-5 in Chinese patent ZL201680037827.7:

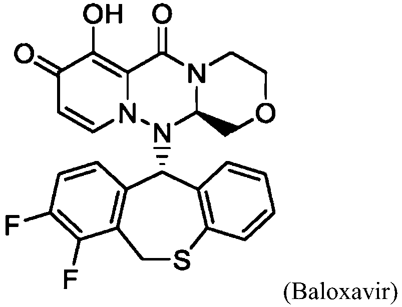

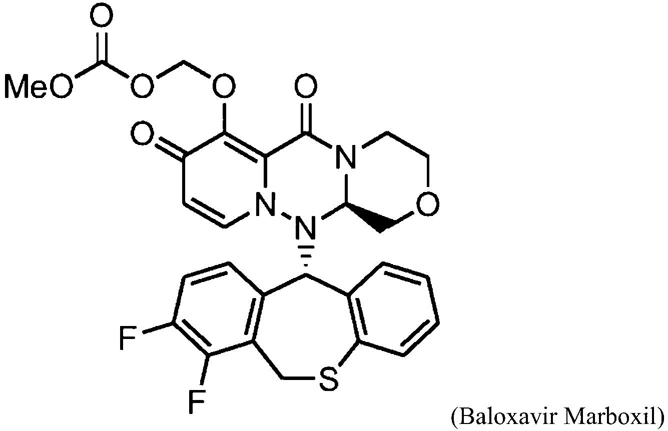

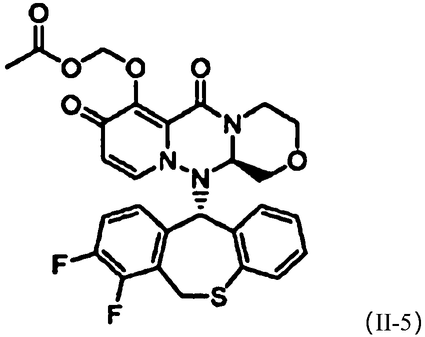

Baloxavir Marboxil is a prodrug of Baloxavir, which has anti-influenza virus activity after metabolism.

Lei Zhao disclosed deuterated Baloxavir Marboxil compounds (e.g., compound I-20) in Chinese patent application (application number) CN201910030450.8, and disclosed anti-influenza activity data of some of these compounds.

Although numerous prodrug compounds of Baloxavir have been disclosed in literature, there is still a desire in the art for anti-viral drugs with high bioavailability, long half-life, and high blood concentration.

### Summary

The present application provides an acyl ethyl ester polycyclic compound and a pharmaceutical composition thereof, which have prophylactic and therapeutic activities against influenza viruses.

In one aspect, the present application provides an acyl ethyl ester polycyclic compound, wherein the acyl ethyl ester polycyclic compound is a compound of Formula (I):

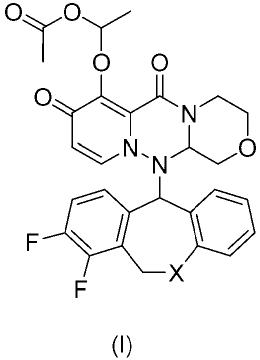

In Formula (I) , X is S (sulfur) or Se (selenium);
or an isomer, a pharmaceutically acceptable salt, a solvate, or an isotope-labeled compound of the compound of Formula (I).

In some embodiments, the compound of Formula (I) is a compound of Formula (1-1): 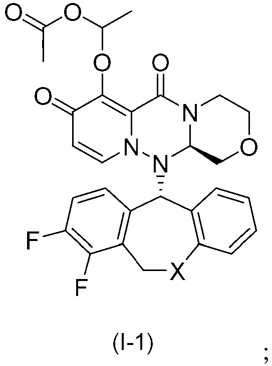 wherein, X is S (sulfur) or Se (selenium).

In some embodiments, the compound of Formula (I) is a compound of Formula (1-1-1):

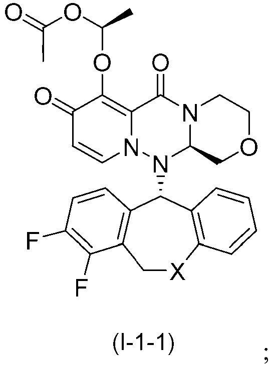

Wherein, X is S (sulfur) or Se (selenium).

In some embodiments, the compound of Formula (I) is a compound of Formula (1-1-2): 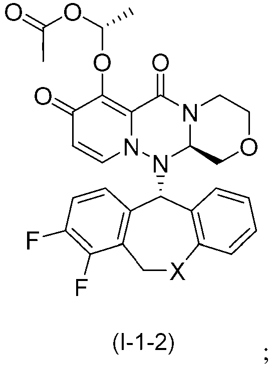 wherein, X is S (sulfur) or Se (selenium).

In some embodiments, in the compound of Formula (I), Formula (1-1), Formula (I-1-1), or Formula (I-1-2), X is S.

In some embodiments, in the compound of Formula (I), Formula (1-1), Formula (I-1-1), or Formula (1-1-2), X is Se.

In an embodiment of the present invention, the compound of Formula (I), Formula (1-1), Formula (I-1-1), or Formula (I-1-2) is optionally in the form of a pharmaceutically acceptable salt, and the "pharmaceutically acceptable salt" refers to a pharmaceutically acceptable acid addition salt.

The "pharmaceutically acceptable acid addition salt" refers to a salt formed with an inorganic acid or an organic acid. Inorganic acid salts include, but are not limited to, hydrochloride, hydrobromide, sulfate, phosphate, and the like. Organic acid salts include, but are not limited to, formate, acetate, propionate, glycolate, gluconate, lactate, oxalate, maleate, succinate, fumarate, tartrate, citrate, glutamate, aspartate, benzoate, methanesulfonate, p-toluenesulfonate, and salicylate, and the like.

In an embodiment of the present invention, the compound of Formula (I), Formula (1-1), Formula (1-1-1), or Formula (I-1-2) may be in the form of a solvate, and the "solvate" refers to a complex formed by a compound of the present invention and a solvent. They react in the solvent or precipitate or crystallize from the solvent. For example, a complex formed with water is referred to as a "hydrate".

In an embodiment of the present invention, a compound of the present invention may comprise one or more chiral centers and can exist in different optically active forms. When the compound comprises one chiral center, the compound comprises enantiomers. The present invention includes both of these enantiomers and mixtures of the enantiomers, such as a racemic mixture. Enantiomers can be resolved by methods known in the art, such as crystallization and chiral chromatography. When the compound of the present invention comprises more than one chiral center, diastereomers may exist. The present invention includes resolved optically pure specific isomers as well as mixtures of diastereomers. Diastereomers can be resolved by methods known in the art, such as crystallization and preparative chromatography.

In an embodiment of the present invention, the compound of Formula (I), Formula (1-1), Formula (1-1-1), or Formula (1-1-2) may be an isotope-labeled compound, wherein the isotopes include atoms having the same atomic number but different mass number. Examples of isotopes that may be incorporated into the compounds of the present invention and pharmaceutically acceptable salts thereof include isotopes of hydrogen, carbon, nitrogen, oxygen, phosphorus, sulfur, fluorine, iodine and chlorine, for example, ²H , ³H, ¹¹C, ¹³C, ¹⁴C, ¹⁵N, ¹⁷O, ¹⁸O, ³¹P, ³²P, ³⁵S, ¹⁸F, ³⁶Cl, ¹²³I, and¹²⁵I. The present invention also includes labeling methods for preparing the isotope label, and the use of the isotope-labeled compound as a metabolic pharmacokinetic study tool and/or a diagnostic tool.

In some embodiments, the compound of Formula (I), Formula (I-1), Formula (I-1-1), or Formula (I-1-2) provided in the present invention is selected from: 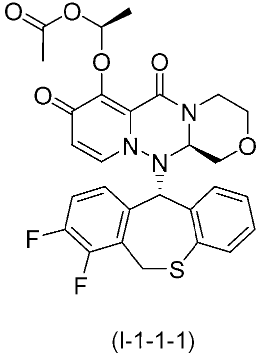 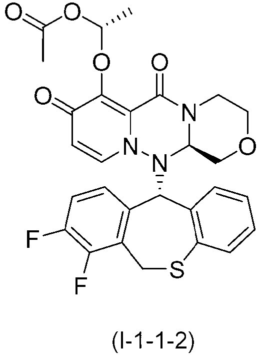 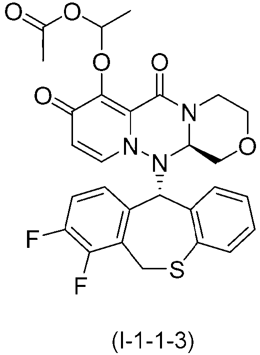 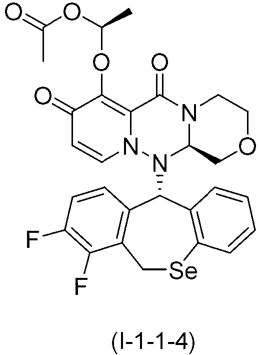 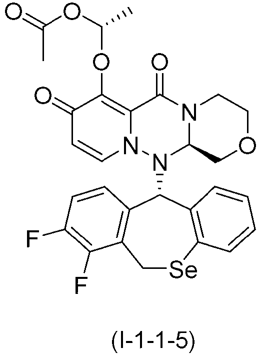 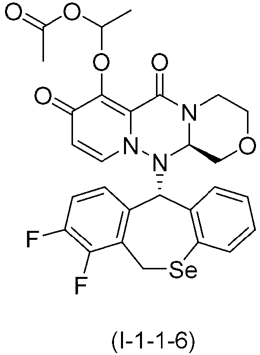 or a pharmaceutically acceptable salt, a solvate, or an isotope-labeled compound thereof.

In yet another aspect, the present invention provides a pharmaceutical composition comprising the compound of Formula (I), Formula (1-1), Formula (I-1-1), or Formula (I-1-2), or a stereoisomer, a pharmaceutically acceptable salt, a solvate, or an isotope-labeled compound thereof. In an embodiment of the present invention, the compound of the present invention in the pharmaceutical composition is present in a therapeutically effective amount.

In some embodiments, the pharmaceutical composition typically comprises a pharmaceutically acceptable carrier, such as a pharmaceutically acceptable diluent, excipient, filler, binder, disintegrant, absorption enhancer, surfactant, lubricant, flavoring agent, sweetening agent, and the like. The pharmaceutical composition can adopt any suitable dosage form, and specifically it can be tablets, powders, capsules, granules, oral liquids, injections, powders, suppositories, pills, creams, pastes, gels, powders (bulk), inhalants, suspensions, dry suspensions, patches, lotions, nanoformulations, and the like.

In some embodiments, the pharmaceutical composition may further comprise one or more therapeutic agents, wherein the therapeutic agent may be selected from neuraminidase inhibitors, nucleoside drugs, PB2 inhibitors, PB1 inhibitors, M2 inhibitors, or other anti-influenza drugs, and the like.

In yet another aspect, the present invention provides the compound of Formula (I), Formula (1-1), Formula (I-1-1), or Formula (I-1-2), or a stereoisomer, a pharmaceutically acceptable salt, a solvate, or an isotope-labeled compound thereof, or a pharmaceutical composition for use in the prevention and/or treatment of a viral infectious disease. Optionally, the viral infectious disease is an infectious disease caused by influenza virus, such as an infectious disease caused by influenza A virus or influenza B virus.

In yet another aspect, the present invention provides the use of the compound of Formula (I), Formula (1-1), Formula (1-1-1), or Formula (1-1-2), or a stereoisomer, a pharmaceutically acceptable salt, a solvate or an isotope-labeled compound thereof, or the pharmaceutical composition in the preparation of a medicament for preventing and/or treating a viral infectious disease. Optionally, the viral infectious disease is an infectious disease caused by influenza virus, such as an infectious disease caused by influenza A virus or influenza B virus.

In yet another aspect, the present invention provides a method for preventing and/or treating a viral infectious disease, wherein the method comprises administering to a subject in need thereof the compound of Formula (I), Formula (1-1), Formula (I-1-1), or Formula (I-1-2), or a stereoisomer, a pharmaceutically acceptable salt, a solvate, or an isotope-labeled compound thereof, or the pharmaceutical composition; optionally, the viral infectious disease is an infectious disease caused by influenza virus, such as an infectious disease caused by influenza A virus or influenza B virus.

### Brief Description of Drawings

FIG. 1 shows the mass spectrum of LC-MS of the compound of Formula (1-1-1-1) of Example 2.
FIG. 2 shows the mass spectrum of LC-MS of the compound of Formula (I-1-1-2) of Example 2.
FIG. 3 shows the mass spectrum of LC-MS of the compound of Formula (I-20) of Comparative Example 1.
FIG. 4 shows the mass spectrum of LC-MS of a compound of Formula (11-5) of Comparative Example 2.
FIG. 5 shows the mass spectrum of LC-MS of the compound of Formula (1-1-1-6) of Example 3.
FIG. 6 shows the single crystal diffraction pattern of the compound of Formula (I-1-1-1) of Example 2.
FIG. 7 shows the rate of change in body weight of mice after administration of each group of drugs.
FIG. 8 shows the survival rate of mice after administration of each group of drugs.
FIG. 9 shows the drug concentration-time profile in SD rats after a single oral gavage administration of different drugs.
FIG. 10 shows the drug concentration-time profile in cynomolgus monkeys after a single oral gavage administration of different drugs.

### Detailed Description

The present invention is further described by examples below. It would be understood to those skilled in the art that equivalent alternate modifications to the following examples using the prior art in light of the teachings of the present invention remain within the scope of the present invention.

### Abbreviation

- ESI: Electrospray ionization
- Tₘₐₓ: Peak time
- Cₘₐₓ: Maximum plasma concentration
- AUC₀₋ₜ: Area under the plasma concentration-time (0-t) curve
- AUC_{0-∞}: Area under the plasma concentration-time (0-∞) curve
- t_{1/2}: Half-life
- DMSO: Dimethyl sulfoxide
- TMS: Tetramethylsilane
- NBS: *N*-Bromosuccinimide
- AIBN: Azobisisobutyronitrile
- TsOH: *p*-Toluenesulfonic acid
- MsOH: Methanesulfonic acid

### Instrument

In the examples of the present invention, the mass spectrometer instrument is: Agilent 6120 Liquid Chromatography-Mass Spectrometer.
Chromatographic column: KROMASIL-C18 (250 × 4. 6 mm, 5 µm)
Mobile phase: 0.1% formic acid-acetonitrile (60: 40)
UV detection wavelength: 254 nm
Flow rate: 1.0 mL/min
Mass Spectrometry: ESI
Nuclear Magnetic Resonance Spectroscopy: Varian INOVA-400;
Solvent: DMSO-d6; Internal standard: TMS

Single crystal ray diffractometer:
Single crystal X-ray diffraction diffractometer with area detector Nonius cad4;
Temperature: 20 °C;
Characteristics and status of testing sample: particles.

### Example 1

500 mg of Baloxavir, 180 mg of 1-bromoethyl acetate, 210 mg of potassium carbonate and 8 mL of dichloromethane were added to a reaction flask, and the reaction was performed at 25 °C for 12 hours. Filtration was performed, and the filtrate was concentrated and then passed through the column with ethyl acetate/*n*-hexane (3/1) to obtain 512 mg of "Compound 1-1-1-3" as a pale yellow solid in 87% yield.

ESI: [M + H]⁺570.2.

¹HNMR (400 MHz, DMSO-d6) δ 7.76-7.63 (m, 0.5H), 7.40 (dd, J = 14.6, 5.8 Hz, 1.5H), 7.23-7.05 (m,3H), 6.99 (dd, J = 16.9, 7.7 Hz, 1H), 6.86 (q, J = 7.8 Hz, 1H), 6.50 (q, J = 5.2 Hz, 0.5H), 6.29 (q, J = 5.3Hz, 0.5H), 5.71 (q, J = 7.3 Hz, 1.5H), 5.54 (s, 0.5H), 5.46-5.36 (m, 1H), 4.46-4.37 (m, 1.5H), 4.23 (t, J = 6.5Hz, 0.5H), 4.04 (t, J = 13.4 Hz, 1.5H), 3.97 (dd, J = 10.6, 3.0 Hz, 0.5H), 3.73-3.62 (m, 1H), 3.47 (t, J = 10.3Hz, 0.5H), 3.31-3.22 (m, 1.5H), 2.93 (q, J = 11.3, 9.1 Hz, 1H), 2.07 (s, 1.5H), 1.94 (s, 1.5H), 1.63 (dd, J = 19.6,5.3 Hz, 3H).

### Example 2

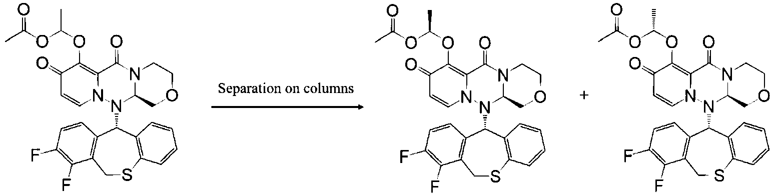

500 mg of "Compound 1-1-1-3" was taken and dissolved in 3 mL of dichloromethane, then mixed with 1.5 g of silica gel. A chromatography column was packed with 20 g of silica gel using ethyl acetate/n-hexane (1/1). And elution was performed with ethyl acetate/n-hexane (1/1) to pass through the column. 165 mg of "Compound 1-1-1-1" and 252 mg of "Compound 1-1-1-2" were obtained as pale yellow solids.

### "Compound 1-1-1":

ESI: [M + H]⁺570.2.

¹H NMR (400 MHz, DMSO-d6) δ 7.46 - 7.36 (m, 2H), 7.16 (d, J = 7.6 Hz, 2H), 7.10 (d, J = 7.9 Hz, 1H), 7.01 (d, J = 7.7 Hz, 1H), 6.85 (t, J = 7.4 Hz, 1H), 6.50 (q, J = 5.2 Hz, 1H), 5.70 (d, J = 7.7 Hz, 1H), 5.54 (s, 1H), 5.46 - 5.37 (m, 1H), 4.46 - 4.38 (m, 2H), 4.06 (d, J = 14.4 Hz, 1H), 3.97 (dd, J = 10.9, 3.1 Hz, 1H), 3.66 (dd, J = 11.8, 3.2 Hz, 1H), 3.47 (t, J = 10.3 Hz, 1H), 3.30 - 3.25 (m, 1H), 3.00 - 2.88 (m, 1H), 2.06 (s, 3H), 1.61 (d, J = 5.3 Hz, 3H).

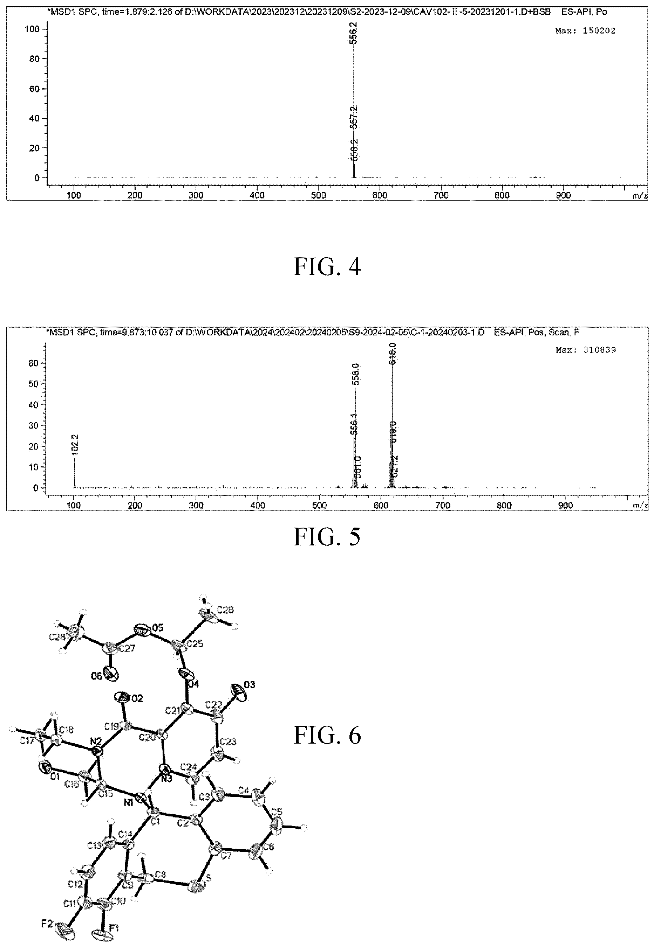

The single crystal diffraction pattern of **Compound I-1-1-1** is shown in FIG. 6. Detection was performed using Cu Ka radiation, with wavelength = 1.54178 A.

According to the analysis of single crystal X-ray diffraction data, the molecular formula of the sample was C₂₈H₂₅F₂N₃O₆S, and the crystal belonged to the monoclinic crystal system with unit cell parameters a = 7.2738 (1) Å, b = 18.5103 (3) Å, c = 10.0095 (2) Å, unit cell volume V = 1300.94 (4) Å³, density Dx = 1.454 mg/m³, Z = 2, F (000) = 592. The molecular structure contained six rings, including one seven-membered ring, three six-membered rings and two benzene rings. The chiral carbon atoms in the structure were consistent with the chirality of the target compound.

### "Compound 1-1-2":

ESI: [M + H]⁺570.2.

¹H NMR (400 MHz, DMSO-d6) δ 7.42 (ddd, J = 15.3, 9.4, 6.4 Hz, 2H), 7.21 - 7.12 (m, 2H), 7.12 - 7.06 (m, 1H), 6.97 (d, J = 7.7 Hz, 1H), 6.87 (t, J = 7.1 Hz, 1H), 6.29 (q, J = 5.3 Hz, 1H), 5.76 - 5.68 (m, 2H), 5.44 - 5.36 (m, 1H), 4.47 - 4.37 (m, 2H), 4.10 - 3.98 (m, 2H), 3.69 (dd, J = 11.5, 3.1 Hz, 1H), 3.35 - 3.21 (m, 2H), 2.98 - 2.86 (m, 1H), 1.93 (s, 3H), 1.66 (d, J = 5.3 Hz, 3H).

### Example 3

**The reaction scheme of Compound C-1 (CompoundI-1-1-6) is as follows:**

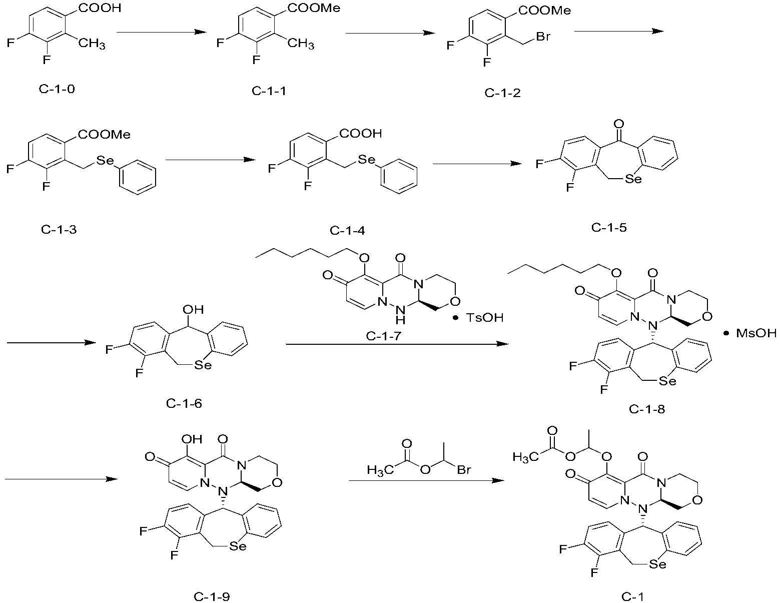

### Example 3-1

20 g of C-1-0, 60 mL of N,N-dimethylacetamide, 22 g of dimethyl sulfate, and 18 g of sodium bicarbonate were added into a reaction flask sequentially. The temperature was raised to 40°C-50°C and the reaction was performed for 4 h. The temperature was cooled down to room temperature. 180 mL of water was added slowly and dropwise. Upon completion of addition, crystallization was performed at room temperature for 3 h. Following filtration, the filter cake was dried under vacuum to obtain 19.1 g of white solid C-1-1 in a yield of 88.7%.

### Example 3-2

7g of C-1-1, 6.65 g ofN-Bromosuccinimide (NBS), 0.77 g of azobisisobutyronitrile (AIBN), and 70 mL of chloroform were added into a reaction flask sequentially. The temperature was raised to 60°C-70°C and the reaction was performed for 4 hours. The temperature was cooled down to room temperature. Following filtration with suction and 10 mL of 10% aqueous sodium sulfite solution was added to the filtrate. Separation was performed, and the aqueous phase was extracted three times with 100 mL of dichloromethane. Following separation, the organic phases were combined. The organic phase was dried with anhydrous sodium sulfate for 1 hour. Filtration was performed, and the filtrate was concentrated under reduced pressure to dryness to obtain 8.67 g of oily substance of C-1-2 in a yield of 87.0%.

### Example 3-3

45 mL of water, 30.5 g of sodium dihydrogen phosphate, 3 g of diphenyl diselenide, and 2.2 g of zinc powder were added into a reaction flask sequentially, stirred at room temperature for 1 hour. 4.48 g of C-1-2 was added and the reaction was performed at room temperature overnight. Filtration was performed with suction, and the filtrate was extracted twice with 100 mL of ethyl acetate. Following separation, the organic phase was dried with anhydrous sodium sulfate for 1 hour. Filtration was performed, and the filtrate was concentrated under reduced pressure to dryness to obtain 5.44 g of oily substance of C-1-3 in a yield of 94.4%.

### Example 3-4

19.15 g of C-1-3, 140 mL of methanol, 7.5 g of sodium hydroxide and 70 mL of water were added into a reaction flask sequentially. The temperature was raised to 60-70 °C and the reaction was performed for 2 hours. Filtration was performed with suction and the filter cake was rinsed twice with 60 mL of water. The pH of the filtrate was adjusted to 7 with 1N hydrochloric acid, and 300 mL of ethyl acetate was added for three extractions. Following separation, the organic phases were combined. The organic phase was dried with anhydrous sodium sulfate for 1 hour. Filtration was performed, and the filtrate was concentrated under reduced pressure to dryness to obtain 12.46 g of solid C-1-4 in a yield of 67.9%.

### Example 3-5

4.7 g of C-1-4 and 98.7 g of polyphosphoric acid were added into a reaction flask sequentially. The temperature was raised to 120 °C and the reaction was performed for 3 hours. The temperature was cooled down to room temperature. The reaction solution was poured into 200 mL of water, and extracted three times with 120 mL of dichloromethane. Following separation, the organic phases were combined. 6.8 g of activated carbon was added to the organic phase for decolorization. Filtration was performed, and the filtrate was concentrated under reduced pressure to dryness to obtain 2.84 g of oily substance of C-1-5 in a yield of 63.9%.

### Example 3-6

2.84 g of C-1-5 and 56.8 g of methanol were added into a reaction flask sequentially, 1.35 g of sodium borohydride was added portionwise. The reaction was performed at room temperature overnight. The pH of the reaction solution was adjusted to 7 with 1 N hydrochloric acid. Then the reaction solution was extracted twice with 80 mL of dichloromethane. Following separation, the organic phases were combined. The organic phase was washed with 40 mL of saturated brine. Separation was performed, and 2 g of anhydrous sodium sulfate was added to the organic phase for drying for 1 hour. Following filtration, the filtrate was concentrated under reduced pressure to obtain a solid of 2.36 g of C-1-6 in a yield of 82.6%.

### Example 3-7

5 mL of ethyl acetate, 5 mL of cyclohexane, 1.88 g of C-1-7, and 1.76 g of C-1-6 were sequentially added into a reaction flask, and stirred for 30 minutes. 4.68 g of 1-n-propylphosphoric anhydride and 0.71 g of methanesulfonic acid were added. The temperature was raised to 60°C-70 °C and maintained, and the reaction was performed for 24 hours. The reaction solution was cooled down to room temperature, and 10 mL of water was added. Extraction was performed with 5 mL of ethyl acetate, and the organic phase was dried with 2 g of anhydrous sodium sulfate for 1 hour. Following filtration, the filtrate was concentrated under reduced pressure to dryness. The residue was dissolved with 5 mL of ethyl acetate and 4 mL of cyclohexane. 0.55 g of methanesulfonic acid was added dropwise. Upon completion of addition, the temperature was controlled at 0-10°C and stirred for 2 hours to effect crystallization. Following filtration, the filter cake was dried under vacuum to obtain a white solid of 1.58 g of C-1-8 in a yield of 58.4%.

### Example 3-8

1.58 g of C-1-8, 0.61 g of lithium chloride, and 5 mL of N-methylpyrrolidone were added into a reaction flask sequentially, the reaction was stirred and the temperature was raised to 75°C-80°C. 0.08 g of methanesulfonic acid was added, and the temperature was maintained and the reaction was performed for 17 hours. The temperature was cooled down to 20°C-30°C, and 10 mL of water was added dropwise while maintaining the temperature. Upon completion of addition, the temperature was cooled down to 5°C-10°C and maintained, stirring for 2 hours. Following filtration, the filter cake was dried under vacuum to obtain 0.84 g of brown compound C-1-9 in a yield of 71.23%.

### Example 3-9

0.8 g of compound C-1-9, 0.4 g of 1-bromoethyl acetate, 0.41 g of potassium carbonate, and 16 mL of acetonitrile were added into a reaction flask sequentially, stirred and the temperature was controlled at 40 °C - 50 °C for reaction for 5 hours. The temperature was cooled down to room temperature. Filtration was performed, and the filter cake was rinsed twice with dichloromethane. The filtrate obtained was passed through the column with ethyl acetate/n-hexane = 1/1. The eluate was concentrated to dryness, and then made into a slurry with 10 mL of n-hexane, filtered to obtain 0.35 g of compound C-1 (compound 1-1-1-6).

ESI: [M + H] ⁺ 618.05, shown in FIG. 5.

1H NMR (400 MHz, DMSO-d6) δ 7.44 - 7.32 (m, 4H), 7.28 - 7.19 (m, 4H), 7.14 - 7.05 (m, 4H), 6.94 - 6.87 (m, 2H), 6.25- 6.50 (m, 2H), 5.85 - 5.62 (m, 3H), 5.59 (s, 1H), 5.28 (ddd, J = 12.2, 9.2, 2.5 Hz, 2H), 4.50 (dt, J = 10.0, 2.9 Hz, 2H), 4.40 (dd, J = 13.7, 3.0 Hz, 2H), 4.10 (d, J = 12.6 Hz, 2H), 4.05 - 4.01 (m, 2H), 3.71-3.64 (m, 2H), 3.51 (d, J = 10.3 Hz, 1H), 3.36 - 3.21 (m, 3H), 2.99 - 2.92 (m, 2H), 2.03 (d, 6H), 1.62 (dd, J = 5.3 Hz, 6H).

### Comparative Example 1

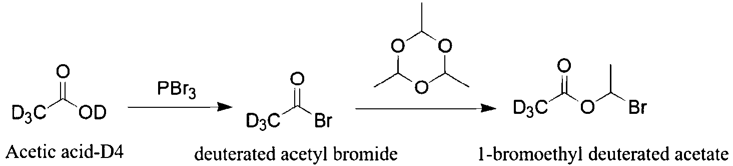

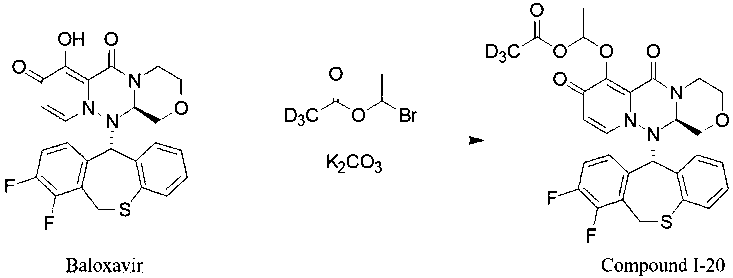

5 mL of phosphorus tribromide was added into a reaction flask, and the temperature was cooled down to 0 °C. 10 g of acetic acid-D4 was added dropwise. Upon completion of addition, the temperature was raised to 45 °C - 50 °C and maintained, and the reaction was performed for 1 hour. The temperature was raised to 75 °C - 80 °C for atmospheric distillation to obtain 12.53 g of oily substance of deuterated acetyl bromide in a yield of 63.74%.

12.5 g of deuterated acetyl bromide and 0.01 g of zinc chloride were added into a reaction flask sequentially, and the temperature was cooled down to -15°C - -10°C. 4.36 g of paraldehyde was added dropwise while controlling the temperature. Upon completion of addition, the temperature was raiesd to 0°C - 10°C and the reaction was performed for 5 hours. 80 mL of water was added dropwise. Upon completion of addition, 50 mL of dichloromethane was added, and extraction was performed. Following separation, the organic phase was then washed with 80 mL of water. Separation was performed, and 2 g of anhydrous sodium sulfate was added into the organic phase for drying for 1 hour. Filtration was performed, and the filtrate was concentrated to dryness under reduced pressure while controlling the temperature at 20 °C - 30 °C to obtain 10.45 g of oily substance of 1-bromoethyl deuterated acetate in a yield of 61.47%.

1.5 g of Baloxavir, 0.85 g of 1-bromoethyl deuterated acetate, 0.86 g of potassium carbonate, 30 mL of acetonitrile were added into a reaction flask sequentially, stirred and the reaction was performed for 15 hours while controlling the temperature at 40 °C - 50°C. The temperature was cooled down to room temperature. Filtration was performed, and the filter cake was rinsed twice with dichloromethane. The filtrate obtained was passed through the column with ethyl acetate/n-hexane = 1/1. The eluent was concentrated to dryness and then made into a slurry with 30 mL of n-hexane, filtered, to obtain 1.2 g of compound I-20.

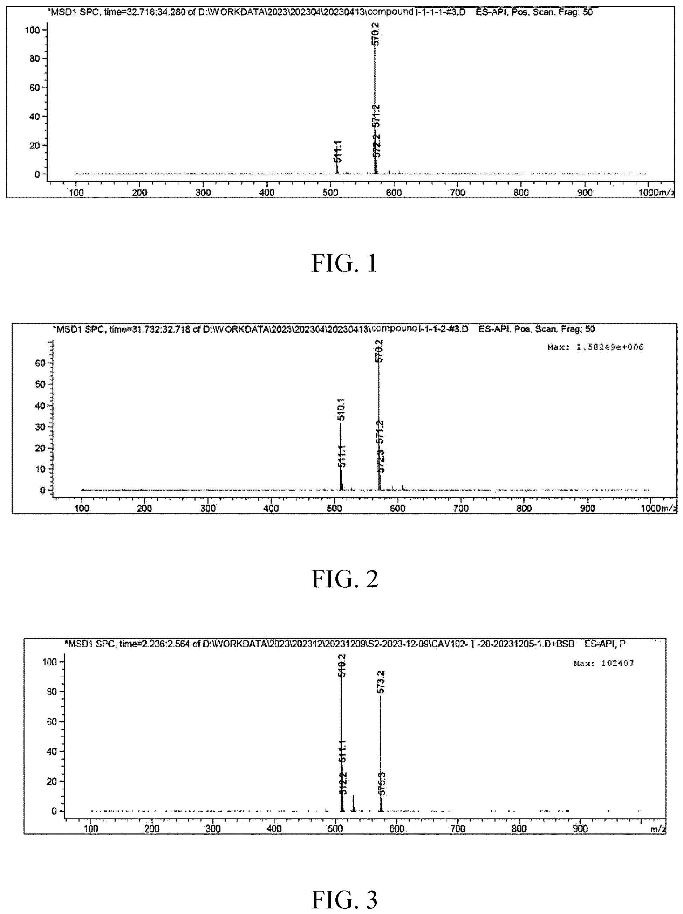

ESI: [M + H]⁺573.2, see Figure 3.

¹H NMR (400 MHz, DMSO-*d*₆) δ 7.46 - 7.37 (m, 4H), 7.18 - 7.08 (m, 6H), 7.00 (ddd, *J =* 15.3, 7.8, 1.5 Hz, 2H), 6.86 (dtd, *J =* 10.3, 7.2, 1.4 Hz, 2H), 6.50 (q, *J =* 5.2 Hz, 1H), 6.28 (q, *J =* 5.2 Hz, 1H), 5.74 - 5.69 (m, 3H), 5.54 (s, 1H), 5.41 (ddd, *J =* 14.6, 7.4, 2.4 Hz, 2H), 4.42 (ddt, *J* = 12.0, 6.4, 2.7 Hz, 4H), 4.08 - 3.95 (m, 4H), 3.68 (ddd, *J =* 14.3, 11.5, 3.2 Hz, 2H), 3.47 (t, *J* = 10.3 Hz, 1H), 3.30 - 3.22 (m, 3H), 2.98 - 2.89 (m, 2H), 1.63 (dd, *J =* 20.1, 5.3 Hz, 6H).

### Comparative Example 2

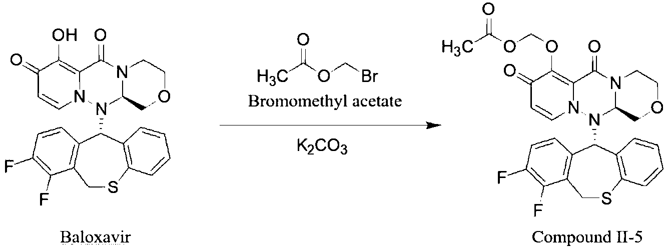

1.5 g of Baloxavir, 0.77 g of bromomethyl acetate, 0.86 g of potassium carbonate and 30 mL of acetonitrile were added into a reaction flask sequentially, stirred and the reaction was performed for 10 hours while controlling the temperature at 40 °C - 50 °C. The temperature was cooled down to room temperature. Filtration was performed, and the filter cake was rinsed twice with dichloromethane. The filtrate obtained was passed through the column with ethyl acetate/n-hexane = 1/1. The eluent was concentrated to dryness and then made into a slurry with 30 mL n-hexane, filtered, to obtain 1.1 g of compound II-5.

ESI:[M+H]⁺556.2, shown in FIG. 4.

¹H NMR (400 MHz, DMSO-*d*₆) δ 7.45 - 7.40(m, 2H), 7.23 (d, *J =* 7.8 Hz, 1H), 7.16 (ddd, *J* = 8.4, 7.1, 1.4 Hz, 1H), 7.09 (dd, *J =* 8.0, 1.4 Hz, 1H), 7.01 (dd, *J =* 7.9, 1.5 Hz, 1H), 6.86 (td, *J =* 7.4, 1.4 Hz, 1H), 5.74 - 5.65 (m, 4H), 5.43 (dd, *J =* 14.3, 2.4 Hz, 1H), 4.43 (td, *J =* 11.2, 10.6, 2.7 Hz, 2H), 4.06 (d, *J =* 14.4 Hz, 1H), 3.99 (dd, *J =* 10.8, 3.1 Hz, 1H), 3.68 (dd, *J =* 11.5, 3.2 Hz, 1H), 3.47 (t, *J =* 10.4 Hz, 1H), 3.32-3.26(m, 1H), 2.94 (ddd, *J =* 14.6, 11.7, 3.4 Hz, 1H), 2.04 (s, 3H).

### Anti-influenza virus activity test in vitro and in vivo:

### 1. Anti-influenza virus activity in vitro

MDCK cells in good logarithmic growth phase were seeded in 96-well plates at 2.5 × 104 cells/well. Except for the normal control group, 100TCID50 infectious amount of H1N1 influenza A virus strain A/FM/1/47 was added to each remaining well and placed in a constant temperature CO₂ incubator for 1 hour. Then serially diluted compound 1-1-1-1, compound 1-1-1-2, compound 1-1-1-3, compound 1-1-1-6, compound I-20, compound 11-5, and Baloxavir Marboxil were added. The cell culture plates with added drugs were continuously incubated at 35 °C for 72 hours. The effect of inhibiting viral replication of the testing drug was determined based on the virus detection results of the supernatant. Finally, the EC₅₀ of the drug in inhibiting virus replication was calculated according to the Reed-Muench method.

The results showed that EC₅₀ (µg/mL) of the compound I-1-1-6, II-5, 1-20, 1-1-1-1, 1-1-1-2, 1-1-1-3, Baloxavir Marboxil for in vitro inhibition of replication of H1N1 (FM1) influenza virus were 0.32, 0.32, 0.32, 0.32, 0.032, 0.32, 0.32, respectively, wherein compound I-1-1-2 showed potent inhibition of replication of H1N1 influenza virus.

### 2. Anti-influenza virus activity in vivo

Female SPF grade ICR mice weighing 19 g -21 g were randomly assigned to 5 groups, with 10 mice in each group. They were blank vehicle group, compound 1-1-1-2 (low dose of 1 mg/kg group and high dose of 5 mg/kg group) and Baloxavir Marboxil (low dose of 1 mg/kg group and high dose of 5 mg/kg group), respectively. A proper amount of FM1 mice lung-adapted H1N1 influenza A strain (A/FM/1/47) was aspirated through a pipette and intranasally inoculated. Administration was started 2 hours after infection and was administered twice daily for 6 consecutive days. The blank vehicle was an aqueous solution of 1% DMSO/4% PEG300/2% Tween 80 (v/v).

During the experiment, the morbidity of animals was observed every day, and the rate change in body weight and death number of mice were recorded. The observation lasted for a total of 15 days, and the survival rate was calculated. Survival rate (%) = number of surviving animals at the end of the experiment/number of animals participating in the experiment × 100%.

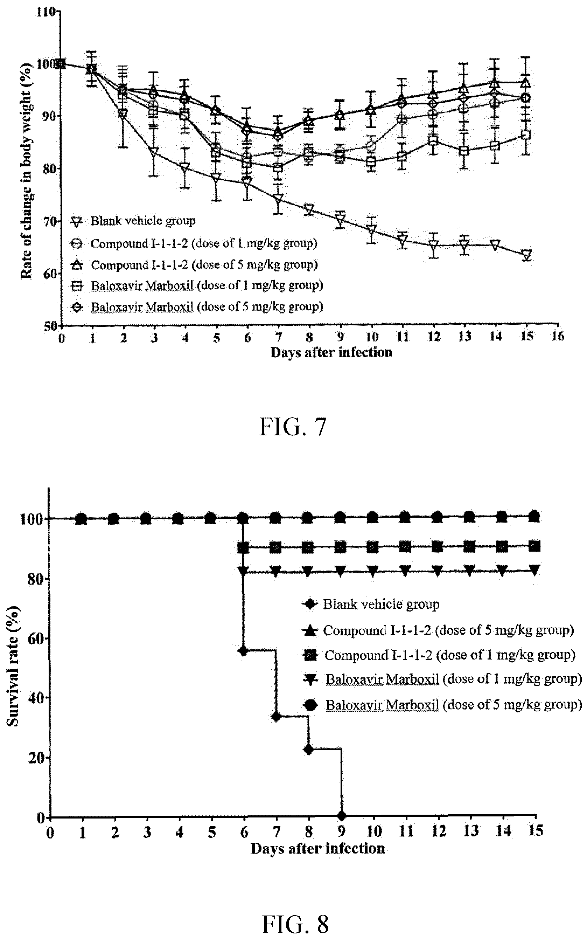

The results shows that: (1) The high dose group of 5 mg/kg of the compound of Formula I-1-1-2 and the high dose group of 5 mg/kg of Baloxavir Marboxil had obvious protective effects on fatal infection caused by H1N1 mice lung-adapted strain, and all mice survived throughout the study, and there was no significant difference in body weight change between the two groups. (2) Compared with the low dose group of 1 mg/kg Baloxavir Marboxil, the mice of the low dose group of 1 mg/kg of Formula 1-1-1-2 had a higher survival rate, and the degree of weight loss of the mice was lower, showing a better protective effect. As shown in FIG. 7 and FIG. 8.

### Biological Test 1-PK Study in Rats

### 1. Study Objectives

SD rats were administered in a single oral gavage administration with different groups of drugs, including compound I-1-1-2, compound 1-1-1-3, compound 1-1-1-6, compound I-20, compound II-5, and Baloxavir Marboxil. After administration, plasma at different time points were collected to detect the drug content, and the pharmacokinetic parameters of different groups of drugs in SD rats were calculated and compared.

### 2. Information of test material, reference material and other vehicle and media

| **2.1** Test material | | |
|---|---|---|
| | **2.1.1** Name: Compound 1-1-1-2 | Batch No.: 20230504-1 |
| | **2.1.2** Name: Compound 1-1-1-3 | Batch No.: 20231227-1 |
| | **2.1.3** Name: Compound 1-1-1-6 | Batch No.: 20240203-1 |
| | **2.1.4** Name: Compound 1-20 | Batch No.: 20231205-1 |
| | **2.1.5** Name: Compound II-5 | Batch No.: 20231201-1 |
| | **2.1.6** Name: Baloxavir Marboxil | Batch No.: BLX-705-220505 |

| **2.2** Vehicle | | |
|---|---|---|
| | **2.2.1** Name: Sodium Lauryl Sulfate (SDS) | Batch No.: 20210426 |
| | **2.2.2** Name: Ethanol | |

### 3. Experimental system

### 3.1 Animal Grouping

48 SD rats, weighing 180 g - 220 g, with half male and half female, were randomly assigned to 6 groups according to their body weight, with 8 rats in each group. Animal grouping information was as follows:

**Table 1 Grouping and identification of SD rats**

| Dose group | Group No. | Gender | Number of animals (number) |
|---|---|---|---|
| Compound 1-1-1-2 | 1 | Male (M) | 4 |
| | | Female (F) | 4 |
| Compound 1-1-1-3 | 2 | Male (M) | 4 |
| | | Female (F) | 4 |
| Compound I-1-1-6 | 3 | Male (M) | 4 |
| | | Female (F) | 4 |
| Compound I-20 | 4 | Male (M) | 4 |
| | | Female (F) | 4 |
| Compound II-5 | 5 | Male (M) | 4 |
| | | Female (F) | 4 |
| Baloxavir Marboxil | 6 | Male (M) | 4 |
| | | Female (F) | 4 |

### 3.2 Administration and sample collection

After SD rats were randomly assigned to groups, they were fasted for more than 12 hours before administration and were allowed to drink water freely. Drinking water was prohibited from 1 hour before administration to 1 hour after administration. Food was provided 4 hours after administration. Each group was administered with a single oral gavage dose of 3.0 mg/kg. Approximately 0.2 mL of jugular vein blood was collected at 0 h before administration and 5 min, 10 min, 15 min, 30 min, 1 h, 2 h, 4 h, 6 h, 10 h, 24 h and 30 h after administration, and placed in EDTA-K2 anticoagulation tubes.

### 3.3 Biological sample analysis

After the whole blood was collected, the plasma was separated by centrifugation at 4000 rpm for 10 min at 4 °C, and then stored in a refrigerator at -80 °C for testing. The plasma drug concentration of samples in each group was determined by LC-MS/MS.

PK parameters were calculated using a non-compartmental model in Phoenix winnonlin 8.0. Data analysis included Cₘₐₓ, Tₘₐₓ, AUC₀₋ₜ, AUC_{0-∞∞}, t_{1/2} and other pharmacokinetic parameters.

### 4. Test Results

### 4.1 Cage-side observation

No obvious abnormalities were found in cage-side observation after administration in all animals.

### 4.2 Test Results

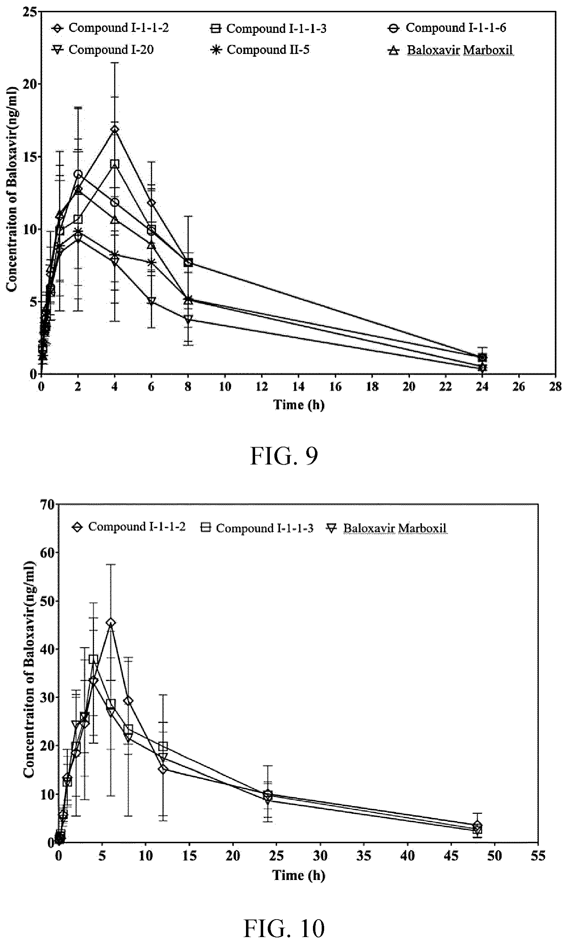

After a single oral gavage administration of the same dose of each group of compounds to SD rats, the main pharmacokinetic parameters were shown in Table 2, and the drug concentration-time profile were shown in FIG. 9.

**Table 2 Pharmacokinetic parameters of SD rats after single oral gavage administration of different drugs**

| Parameter | Group | | | | | |
|---|---|---|---|---|---|---|
| | Compound I-1-1-2 | Compound I-1-1-3 | Compound I-1-1-6 | Compound I-20 | Compound II-5 | Baloxavir Marboxil |
| Tₘₐₓ (hr) | 3.51±1.8 | 4.74±0.9 | 3.2±1.3 | 4.68±1.2 | 4.23±0.9 | 2.2±1.1 |
| Cₘₐₓ (ng/mL) | 17.3±4.7**^{###} | 15.1±4.2*^{##} | 14.6±5.4^{##} | 10.8±3.2 | 10.6±2.8 | 12.6±6.8^{#} |
| AUC₀₋ₜ (hr * ng/mL) | 154±43.8**^{###} | 122±29.7*^{##} | 118±24.3^{##} | 84.6±24.6 | 75.8±29.5 | 108±33.3^{#} |
| AUC_{0-∞} (hr * ng/mL) | 166±43.0 | 125±29.8 | 121±38.5 | 90.4±33.5 | 82.6±26.7 | 117±34.7 |
| t_{1/2} (hr) | 6.86±3.6 | 6.17±2.7 | 5.54±1.1 | 4.74±0.9 | 4.23±0.9 | 6.02±2.6 |

| | | | | | | |
|---|---|---|---|---|---|---|
| Note: (1) Compared with Baloxavir Marboxil group, ***P*<0.01; **P*<0.05. (2) Compared with compound 1-20 and compound II-5, *^{###}P<*0.001; *^{##}P<*0.01; *^{#}P<*0.05*.* | | | | | | |

### Study results:

(1) After a single oral gavage administration of the same dose of compounds to SD rats, the bioavailability (Cₘₐₓ and AUC₀₋ₜ) of rats in each group were sorted in descending order as: Compound I-1-1-2 > Compound I-1-1-3 > Compound I-1-1-6 > Baloxavir Marboxil > Compound I-20 > Compound II-5.
(2) Compared with Compound I-20 and Compound 11-5 groups, the bioavailability (Cₘₐₓ and AUC₀₋ₜ) of rats in Compound 1-1-1-2, Compound 1-1-1-3 and Baloxavir Marboxil groups were significantly improved, with statistical differences (P<0.001, P<0.01 and P<0.05, respectively).
(3) Compared with the Baloxavir Marboxil group, the Cₘₐₓ and AUC₀₋ₜ of the bioavailability of rats in compound I-1-1-2 group and compound 1-1-1-3 group were significantly improved, with statistical difference (P<0.01 and P<0.05).
(4) Compound 1-1-1-2 group had the longest half-life (t_{1/2}), followed by the compound I-1-1-3 group, Baloxavir Marboxil group, and compound 1-1-1-6 group. It suggested that these groups of compounds had the potential to maintain their efficacy for a long time.

### Biological test 2-PK study in cynomolgus monkeys

### 1. Study Objectives

Cynomolgus monkeys were administered with a single oral gavage with different groups of drugs, including compound 1-1-1-2, compound I-1-1-3 and Baloxavir Marboxil. After administration, plasma at different time points were collected to detect the drug content, and the pharmacokinetic parameters of different groups of drugs in cynomolgus monkeys were calculated and compared.

### 2. Information of test material, reference material and other vehicle and media

| **2.1** Test material | | |
|---|---|---|
| | **2.1.1** Name: Compound 1-1-1-2 | Batch No.: 20230504-1 |
| | **2.1.2** Name: Compound 1-1-1-3 | Batch No.: 20231227-1 |
| | **2.1.3** Name: Baloxavir Marboxil | Batch No.: BLX-705-220505 |

| **2.2** Vehicle | | |
|---|---|---|
| | **2.2.1** Name: Sodium Lauryl Sulfate (SDS) | Batch No.: 20210426 |
| **2.2.2** Name: Ethanol | | |

### 3. Experimental system

### 3.1 Animal Grouping

24 cynomolgus monkeys, aged 3-5 years old and weighing 3 kg - 7 kg, with half male and half female, were used and randomly assigned to 3 groups according to their body weight, with 8 monkeys in each group. Animal grouping information was as follows:

**Table 3 Grouping and identification of cynomolgus monkeys**

| Dose group | Group No. | Gender | Number of animals (number) |
|---|---|---|---|
| Compound 1-1-1-2 | 1 | Male (M) | 4 |
| | | Female (F) | 4 |
| Compound 1-1-1-3 | 2 | Male (M) | 4 |
| | | Female (F) | 4 |
| Baloxavir Marboxil | 3 | Male (M) | 4 |
| | | Female (F) | 4 |

### 3.2 Administration and sample collection

After cynomolgus monkeys were randomly assigned to groups, they were fasted for more than 12 hours before administration and were allowed to drink water freely. Drinking water was prohibited from 1 hour before administration to 1 hour after administration. Food was provided 4 hours after administration. Each group was administered with a single oral gavage dose of 5.0 mg/kg. Approximately 1.0 mL of jugular vein blood was collected at 0 h before administration and 5 min, 10 min, 15 min, 30 min, 1 h, 2 h, 4 h, 6 h, 8 h, 12 h, 24 h, and 48 h (D2) after administration, and placed in EDTA-K2 anticoagulation tubes.

### 3.3 Biological sample analysis

After the whole blood was collected, the plasma was separated by centrifugation at 4000 rpm for 10 min at 4 °C, and then stored in a refrigerator at -80 °C for testing. The plasma drug concentration of samples in each group was determined by LC-MS/MS.

PK parameters were calculated using non-compartmental model in a Phoenix winnonlin 8.0. Data analysis included Cₘₐₓ, Tₘₐₓ, AUC₀₋ₜ, AUC,_{0-∞∞}, t_{1/2} and other pharmacokinetic parameters.

### 4. Test Results

### 4.1 Cage-side observation

No obvious abnormalities were found in cage-side observation after administration in all animals.

### 4.2 Test Results

After a single oral gavage administration of the same dose of each group of compounds to cynomolgus monkeys, the main pharmacokinetic parameters were shown in Table 4, and the drug concentration-time profile were shown in Fig. 10.

**Table 4 Pharmacokinetic parameters of cynomolgus monkeys after a single oral gavage administration of different groups of drugs**

| Parameter Name | Group | | |
|---|---|---|---|
| | Compound I-1-1-2 | Compound 1-1-1-3 | Baloxavir Marboxil |
| Tₘₐₓ (hr) | 4.83±1.3 | 5.33±3.8 | 2.5±0.8 |
| Cₘₐₓ (ng/mL) | 45.9±24.7** | 38.1±15.1* | 33.3±16.6 |
| AUC₀₋ₜ (hr * ng/mL) | 593±335.1 | 491±404.1 | 402±178.2 |
| AUC_{0-∞} (hr * ng/mL) | 700±369.2 | 576±483.9 | 493±146.3 |
| t_{1/2} (hr) | 17.1±7.3 | 13.4±1.9 | 12.4±1.7 |

| | | | |
|---|---|---|---|
| Note: Compared with Baloxavir Marboxil group, ***P*<0.01; **P*<0.05. | | | |

### Study results:

(1) After a single oral gavage administration of the same dose of compounds to cynomolgus monkeys, the bioavailability (Cₘₐₓ and AUC₀₋ₜ) of cynomolgus monkeys in each group were sorted in descending order as: Compound I-1-1-2 > Compound I-1-1-3 > Baloxavir Marboxil.
(2) Compared with the Baloxavir Marboxil group, the bioavailability (Cₘₐₓ and AUC₀₋ₜ) of Compound 1-1-1-2 group and Compound 1-1-1-3 group significantly improved, with statistical differences (P<0.01 and P<0.05, respectively).
(3) Compound 1-1-1-2 group had the highest exposure and the longest half-life in cynomolgus monkeys. The drug therapeutic effect could be maintained for a long time.

## Claims

1. An acyl ethyl ester polycyclic compound, wherein the acyl ethyl ester polycyclic compound is a compound of Formula (I): 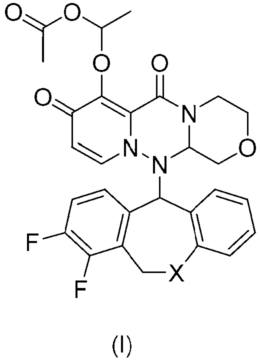
in Formula (I), X is S (sulfur) or Se (selenium);
or an isomer, a pharmaceutically acceptable salt, a solvate, or an isotope-labeled compound of the compound of Formula (I).

2. The acyl ethyl ester polycyclic compound of claim 1, wherein the compound of Formula (I) is a compound of Formula (1-1):

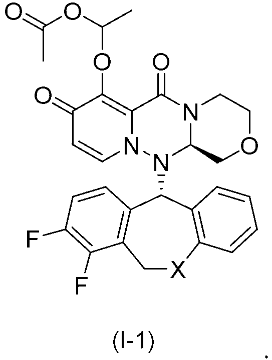

3. The acyl ethyl ester polycyclic compound of claim 2, wherein the compound of Formula (I) is a compound of Formula (1-1-1):

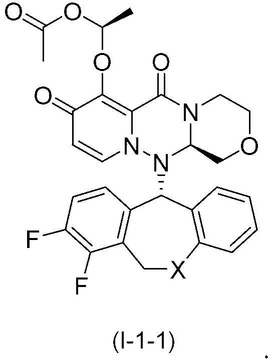

4. The acyl ethyl ester polycyclic compound of claim 2, wherein the compound of Formula (I) is a compound of Formula (I-1-2):

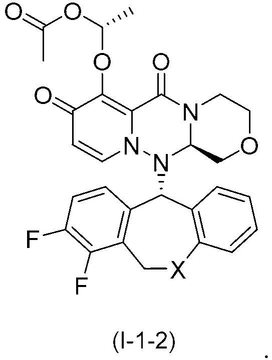

5. The acyl ethyl ester polycyclic compound of any one of claims 1 to 4, wherein X is S.

6. The acyl ethyl ester polycyclic compound of any one of claims 1 to 4, wherein X is Se.

7. A compound selected from: 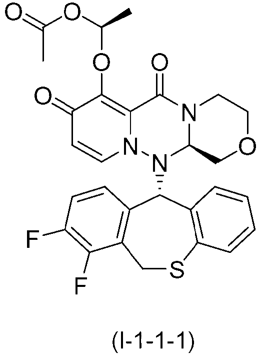 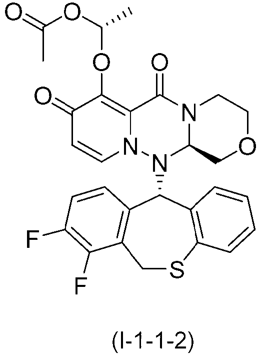 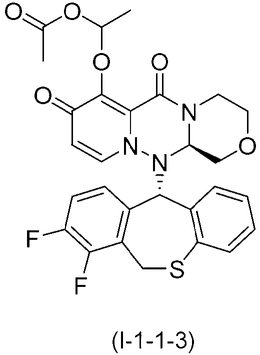 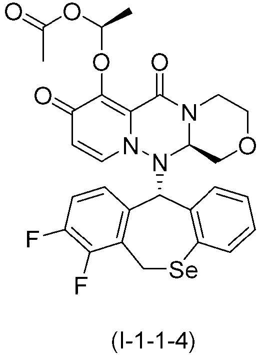 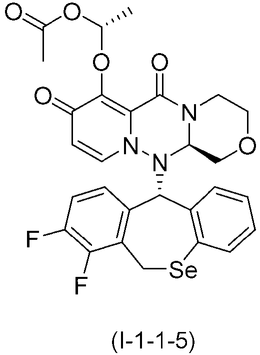 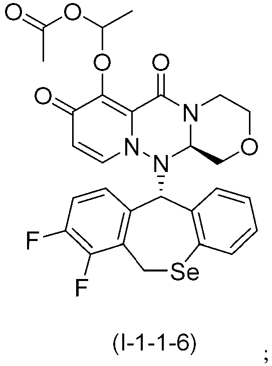 or a pharmaceutically acceptable salt, a solvate, or an isotope-labeled compound thereof.

8. A pharmaceutical composition comprising the compound of any one of claims 1 to 7.

9. Use of the compound of any one of claims 1 to 7 or the pharmaceutical composition of claim 8 for the preparation of a medicament for preventing and/or treating of a viral infectious disease, optionally, the viral infectious disease is an infectious disease caused by influenza A virus or influenza B virus.

10. The compound of any one of claims 1 to 7 or the pharmaceutical composition of claim 8 for use in preventing and/or treating a viral infectious disease, optionally, the viral infectious disease is an infectious disease caused by influenza A virus or influenza B virus.

11. A method of preventing and/or treating a viral infectious disease, wherein the method comprises administering to a subject in need thereof the compound of any one of claims 1 to 7 or the pharmaceutical composition of claim 8, optionally, the viral infectious disease is an infectious disease caused by influenza A virus or influenza B virus.
